# EUROPÄISCHE PATENTANMELDUNG

(11) **EP 4 844 395 A1**
(43) Veröffentlichungstag der Anmeldung: **25.11.2026**
(21) Anmeldenummer: 26179273.3
(22) Anmeldetag: 18.05.2026
(51) Int. Cl.: G01N 29/036, F24F 1/16, F24F 11/49, G01N 29/02, G01N 29/22, G01N 29/32, G01N 33/18

(54) **FLÜSSIGKEITSRÜCKHALTESYSTEM**

(30) Priorität: 23.05.2025 DE 102025120202
(71) Anmelder: GUS Gewässer-Umwelt-Schutz GmbH, 48531 Nordhorn (DE)
(72) Erfinder: Junge, Johannes, 48531 Nordhorn (DE); Sobiella, Patrick, 29313 Hambühren (DE); Krenn, Christoph, 29313 Hambühren (DE)
(74) Vertreter: Holz, Christian

(57) **Zusammenfassung**

Die vorliegende Erfindung betrifft ein Flüssigkeitsrückhaltesystem (2) mit einer Auffangwanne (20), welche ausgebildet ist, einen chemischen Stoff einer Anlage (1) und ein wässriges Medium (3) gemeinsam aufzufangen, und mit einem betätigbaren Auslass (24), vorzugsweise einem Ventil (24), welcher ausgebildet ist, ein Abfließen des den chemischen Stoff der Anlage (1) enthaltenden wässrigen Mediums (3) aus der Auffangwanne (20) zuzulassen oder zu unterbinden, wobei das Flüssigkeitsrückhaltesystem (2), vorzugsweise eine Steuerungseinheit (26) des Flüssigkeitsrückhaltesystems (2) oder eine ein Schwingungselement aufweisende Sensoreinheit 10 (25) des Flüssigkeitsrückhaltesystems (2), ausgebildet und eingerichtet ist:
• ein Schwingungselement, vorzugsweise eine Schwinggabel, welches in Kontakt mit dem den chemischen Stoff der Anlage enthaltenden wässrigen Medium steht, mit seiner Resonanzfrequenz anzuregen,
• eine Resonanzfrequenz des Schwingungselements zu erfassen,
• den Gehalt des chemischen Stoffes der Anlage (1) im wässrigen Medium (3) basierend auf der erfassten Resonanzfrequenz des Schwingungselements zu bestimmen und
• den betätigbaren Auslass (24) in Abhängigkeit des bestimmten Gehalts des chemischen Stoffes der Anlage (1) im wässrigen Medium (3) zu öffnen oder zu schließen.

## Beschreibung

Die vorliegende Erfindung betrifft ein Flüssigkeitsrückhaltesystem sowie eine Anlage mit einem derartigen Flüssigkeitsrückhaltesystem.

Bei verschiedenen technischen Anlagen bzw. Anwendungen werden Glykole, auch Glycole geschrieben, eingesetzt. Glykole dienen insbesondere als Kühlmittel zum Abtransport von Wärme, aber auch als Gefrierschutzmittel, um den Gefrierpunkt eines anderen chemischen Stoffes wie z. B. Wasser herabzusetzen. Gefrierschutzmittel werden umgangssprachlich auch als Frostschutzmittel bezeichnet. Zu den Glykolen gehören beispielsweise Propylenglykol sowie Ethylenglykol. Es können jedoch auch andere chemische Stoffe als Glykol als Gefrierschutzmittel eingesetzt werden.

Da Gefrierschutzmittel und insbesondere Glykole toxisch sind, ist es beispielsweise in der Kälte- und Klimaanlagentechnik vorgeschrieben, den Austritt des Gefrierschutzmittels zu erkennen und Maßnahmen einzuleiten, um den Austritt des Gefrierschutzmittels zu unterbinden sowie das ausgetretene Gefrierschutzmittel aufzufangen und von der Umgebung bzw. von der Umwelt wie insbesondere vom Untergrund und damit vom Grundwasser fernzuhalten.

Bekannt ist es daher, unterhalb der Kälte- oder Klimaanlage eine Auffangwanne, ein Auffangbecken bzw. ein Rückhaltebecken vorzusehen, welches aus der Kälte- oder Klimaanlage unabsichtlich austretendes Gefrierschutzmittel als chemischen Stoff der Anlage auffangen kann, so dass das Gefrierschutzmittel nicht in die Umgebung wie insbesondere in den Untergrund gelangen kann.

Befindet sich die Kälte- oder Klimaanlage im Freien, was zur Verbesserung der wärmetauschenden Wirkung mit der Umgebungsluft üblich ist, so kann auch Regenwasser aus der Umgebung sowie Kondenswasser, welches sich an der Außenseite der Kälte- oder Klimaanlage bilden kann, in die Auffangwanne tropfen und sich dort mit dem dort aufgefangenen Gefrierschutzmittel vermischen. Dieses zusätzliche Wasser, d.h. Regenwasser und bzw. oder Kondenswasser, kann die Auffangwanne ggfs. zum Überlaufen bringen, wodurch ebenfalls in der Auffangwanne befindliches bzw. aufgefangenes Gefrierschutzmittel als chemischer Stoff der Anlage mit dem Regenwasser und bzw. oder Kondenswasser zusammen als wässriges Medium doch in die Umgebung und insbesondere in den Untergrund, ggfs. ins Grundwasser, gelangen kann.

Daher ist es zum einen üblich, bei Kälte- oder Klimaanlagen einen Wannenablauf vorzusehen, über welchen das aufgefangene Regen- bzw. Kondenswasser als wässriges Medium aus der Auffangwanne kontrolliert bzw. gezielt ins Abwasser abgeführt werden kann. Zum anderen ist es üblich, eine geeignete Sensorik vorzusehen, welche Gefrierschutzmittel im aufgefangenen bzw. im ablaufenden Regenwasser und bzw. oder Kondenswasser erfassen bzw. erkennen kann. Somit ist es bekannt, den Anteil bzw. den Gehalt von Gefrierschutzmittel als chemischer Stoff im aufgefangenen bzw. im ablaufenden Regenwasser und bzw. oder Kondenswasser als den chemischen Stoff der Anlage enthaltendes wässriges Medium einer Kälte- oder Klimaanlage zu erfassen, indem ein entsprechender Sensor im Bereich des Wannenablaufs vom ablaufenden vermischten wässrigen Medium passiert und dabei der Anteil bzw. den Gehalt von Gefrierschutzmittel im ablaufenden Regenwasser und bzw. oder Kondenswasser sensorisch erfasst wird. Ein derartiger Sensor bzw. eine derartige Sensorik kann in den automatisch ablaufenden Prozess der Kälte- oder Klimaanlage eingebunden sein und daher als In-Line-Sensorik bezeichnet werden.

Wird bei dieser Messung ein, beispielsweise vom Anlagenbetreiber vorbestimmter, Grenzwert der prozentualen Gefrierschutzmittelkonzentration wie insbesondere der prozentualen Glykolkonzentration überschritten, erfolgt seitens der Anlagen bzw. seitens der Anlagensteuerung selbsttätig ein Schließen eines Absperrventils des Wannenablaufs, so dass das unzulässig viel Gefrierschutzmittel bzw. Glykol enthaltende vermischte wässrige Medium von der Umwelt und insbesondere vom Grundwasser ferngehalten und behandelt bzw. entsorgt werden kann. Das Schließen des Ventils kann ggfs. gemeldet werden, um Wartungspersonal zu benachrichtigen. Dies kann die Verwendung von Gefrierschutzmittel und insbesondere von Glykol, insbesondere für Kälte- oder Klimaanlagen, ermöglichen und gleichzeitig das Gefrierschutzmittel und insbesondere das Glykol von der Umwelt und insbesondere vom Grundwasser fernhalten.

Um insbesondere Glykolanteile im vermischten wässrigen Medium insbesondere der Auffangwanne einer Kälte- oder Klimaanlage erkennen zu können, sind verschiedene Messmethoden allgemein bekannt.

Hierzu gehört die Leitwertmessung, um die elektrische Leitfähigkeit des wässrigen Mediums zu erfassen bzw. zu messen und hieraus auf den Glykolanteil im wässrigen Medium zu schließen. Nachteilig ist hierbei jedoch, dass die Messung beispielsweise durch Verschmutzungen oder durch im wässrigen Medium gelöste Salze stark verfälscht werden kann.

Auch ist die Messung des PH-Wertes des wässrigen Mediums bekannt, um den PH-Wert des wässrigen Mediums zu erfassen bzw. zu messen und hieraus auf den Glykolanteil im wässrigen Medium zu schließen. Nachteilig hierbei ist jedoch der hoher Wartungsaufwand aufgrund der sehr kurzen Kalibrierzyklen. Auch darf die Sonde nicht austrocknen. Die Messung des PH-Wertes des wässrigen Mediums stellt somit kein geeignetes In-Line Messverfahren dar.

Ferner ist die Refraktometrie bekannt, um den Brechungsindex des wässrigen Mediums zu erfassen bzw. zu messen und hieraus auf den Glykolanteil im wässrigen Medium zu schließen. Nachteilig ist hierbei jedoch, dass das Messprinzip empfindlich auf eine Verschmutzung des Prismas reagiert, weshalb ein In-Line Messverfahren mit erhöhtem Aufwand wie beispielsweise einer automatischen Ultraschallreinigung oder einer manuellen Reinigung mit kurzen Reinigungsintervallen verbunden ist.

Des Weiteren ist die Dichtemessung mittels Biegeschwinger-Messung bekannt, indem die Dichte des wässrigen Mediums aus der Messung der Veränderung der Resonanzfrequenz des Biegeschwingers, welche durch die Masse des im Biegeschwinger-Röhrchen enthaltenen Mediums bei bekanntem Volumen hervorgerufen wird, errechnet bzw. bestimmt wird. Nachteilig ist in diesem Fall, dass bereits durch geringe grobe Verschmutzungen wie beispielsweise Partikel, Schwebestoffe und dergleichen im wässrigen Medium die Messung verfälscht werden kann. Dies kann durch die Verwendung eines Bypasses und eines Feinfilters reduziert werden, was jedoch einen hohen Wartungsaufwand verursachen kann. Auch besteht die Gefahr, dass bei verstopftem Filter austretendes Glykol nicht erkannt werden kann. Ferner muss ein Gefrieren des wässrigen Mediums im Biegeschwinger verhindert werden, was mittels einer Begleitheizung erreicht werden kann. Dies erhöht jedoch den Aufwand weiter. Auch kann das Schwingelement beschädigt werden, falls die Begleitheizung ausfällt.

Darüber hinaus ist auch die Dichtemessung mittels Coriolis-Massendurchflussmesser bekannt, indem der Massenstrom vom durchströmenden wässrigen Medium gemessen wird. Das Messverfahren beruht auf dem Coriolis-Prinzip. Nachteilig ist auch in diesem Fall, dass die Messung bereits durch geringe grobe Verschmutzungen wie beispielsweise Partikel, Schwebestoffe und dergleichen im wässrigen Medium verfälscht werden kann. Ferner kann auch in diesem Fall ein Bypass und ein Feinfilter notwendig sein, mit den zuvor beschriebenen weiteren hierdurch entstehenden Nachteilen. Außerdem ist bei diesem Messprinzip ein Durchfluss des wässrigen Mediums durch den Coriolis-Massendurchflussmesser zwingend erforderlich, d.h. die Dichtemessung mittels Coriolis-Massendurchflussmesser ist nur bei strömenden wässrigen Medien aber nicht bei stehenden wässrigen Medien anwendbar.

Die EP 3 301 440 A1 beschreibt ein Flüssigkeitsrückhaltesystem mit zumindest einer unter einem Aggregat einer leckagegefährdeten Anlage platzierten Auffangwanne und einem Ultraschallsensor, wobei mittels des Ultraschallsensors ein Sensorsignal generierbar ist, welches eine Erkennung von Glykol in einem Wasser-Glykol-Gemisch, dem der Ultraschallsensor ausgesetzt ist, kodiert und wobei mittelbar oder unmittelbar aufgrund des Sensorsignals ein stromabwärts der Auffangwanne platziertes Ventil ansteuerbar ist.

Eine Aufgabe der vorliegenden Erfindung ist es, die Möglichkeiten zu verbessern und bzw. oder zu erweitern, den Anteil eines chemischen Stoffes, insbesondere von Gefrierschutzmittel, ganz besonders von Glykol, einer Anlage in einem wässrigen Medium eines Flüssigkeitsrückhaltesystem sensorisch zu erfassen. Dies soll möglichst im Rahmen eines In-Line Messverfahrens erfolgen bzw. genutzt werden können. Insbesondere soll der Wartungsaufwand des entsprechenden Sensors gegenüber vergleichbaren bekannten Sensoren reduziert werden. Insbesondere soll die Robustheit des entsprechenden Sensors hinsichtlich grober Verschmutzungen gegenüber vergleichbaren bekannten Sensoren erhöht werden. Insbesondere soll die Notwendigkeit der Filterung des wässrigen Mediums gegenüber vergleichbaren bekannten Sensoren reduziert werden. In jedem Fall soll dies insbesondere sowohl auf fließende als auch auf stehende wässrige Medien anwendbar sein. Zumindest soll eine Alternative zu den bekannten Möglichkeiten geschaffen werden.

Die Aufgabe wird erfindungsgemäß durch ein Flüssigkeitsrückhaltesystem mit den Merkmalen des Patentanspruchs 1 gelöst. Vorteilhafte Weiterbildungen sind in den Unteransprüchen beschrieben.

Somit betrifft die vorliegende Erfindung ein Flüssigkeitsrückhaltesystem mit einer Auffangwanne, welche ausgebildet ist, einen chemischen Stoff einer Anlage und ein wässriges Medium gemeinsam aufzufangen. Die Auffangwanne kann auch als Auffangbecken oder als Rückhaltebecken bezeichnet werden. Die Auffangwanne kann aus Metall, aus Kunststoff oder aus Keramik ausgebildet und in jedem Fall chemisch gegenüber dem aufzufangenden chemischen Stoff der Anlage beständig sein, ferner gegenüber Umwelteinflüssen wie Hitze und Frost, Sonneneinstrahlung und bzw. oder Eis, Schnee, Hagel und dergleichen.

Der besagte chemische Stoff der Anlage kann ein jeglicher chemischer Stoff sein, welcher in der entsprechenden Anlage zur Durchführung des entsprechenden Prozesses verwendet werden oder bei dem Prozess entstehen kann. Der chemische Stoff der Anlage kann insbesondere flüssig oder zumindest in Flüssigkeiten und insbesondere in Wasser löslich sein. In jedem Fall ist ein Austritt des chemischen Stoffes aus der Anlage unabsichtlich und wird beispielsweise durch zumindest langfristig auftretende und unvermeidbare Undichtigkeiten der Anlage verursacht, welche mit der Zeit aufgrund von Materialermüdung von Dichtungen und dergleichen entstehen können. Dies kann insbesondere der Fall sein, falls der chemische Stoff der Anlage unter Druck steht, wie dies beispielweise bei einem Gefrierschutzmittel einer Kälte- oder Klimaanlage der Fall ist.

Der chemische Stoff der Anlage kann insbesondere ein Gefrierschutzmittel einer Kälte- oder Klimaanlage sein, wobei insbesondere die Verwendung von Glykol als Gefrierschutzmittel üblich sein kann. Die vorliegende Erfindung lässt sich jedoch auch auf andere chemische Stoffe von Anlagen anwenden, welche ebenso wie das ungewollt austretende Gefrierschutzmittel einer Kälte- oder Klimaanlage aufzufangen und von der Umwelt bzw. von der Umgebung und insbesondere um Untergrund bzw. vom Grundwasser fernzuhalten sind. Dies kann ebenso für mehrere chemische Stoffe der Anlage bzw. für Gemische aus chemischen Stoffen der Anlage gelten, welche gemeinsam aufzufangen und von der Umwelt fernzuhalten sind. In jedem Fall kann der chemische Stoff der Anlage bzw. können die chemischen Stoffe der Anlage, welche aufzufangen sind, toxisch bzw. für die Umwelt und insbesondere für Menschen und bzw. oder Tiere schädlich sein.

In jedem Fall kann das wässrige Medium ein weiterer chemischer Stoff sein, welcher jedoch nicht der Anlage entstammt bzw. nicht in der Anlage verwendet wird sowie welcher an sich unbedenklich ist und lediglich durch die Vermischung mit dem zuvor beschrieben aufzufangenden chemischen Stoff der Anlage ebenfalls von der Umwelt fernzuhalten sein kann. Insbesondere kann dieser weitere unbedenkliche bzw. harmlose chemische Stoff Wasser sein, welches insbesondere als Regenwasser und bzw. oder als Kondenswasser zusätzlich zum chemischen Stoff der Anlage bzw. ungewollt von der Auffangwanne aufgefangen werden kann, weil die Auffangwanne gegenüber der Umgebung und insbesondere nach oben hin offen ausgebildet sein kann, wodurch Regen in die Auffangwanne gelangen kann und bzw. oder gegenüber Flächen der Anlage offen ausgebildet ist, an welchen sich Kondenswasser bilden und in die Auffangwanne laufen und bzw. oder tropfen kann.

Werden nun der chemische Stoff der Anlage sowie der flüssige chemische Stoff, welcher aufgrund seines größeren bzw. wesentlichen Anteils als wässriges Medium bezeichnet werden kann, gemeinsam von der Auffangwanne aufgefangen bzw. aufgenommen, erfolgt eine Vermischung zu einem den chemischen Stoff der Anlage enthaltenden wässrigen Medium. Das wässrige Medium, welches vorzugsweise Wasser in Form von Regenwasser und bzw. oder von Kondenswasser sein kann, kann somit einen gewissen Gehalt bzw. Anteil des chemischen Stoffs der Anlage aufweisen, welcher bedenklich bzw. toxisch sein kann. Tritt kein bedenklicher chemischer Stoff aus der Anlage aus und wird deshalb nicht von der Auffangwanne aufgefangen, so kann der Gehalt des chemischen Stoffs der Anlage auch Null betragen, wenngleich im Folgenden die Formulierung des "den chemischen Stoff der Anlage enthaltenden wässrigen Mediums" sowohl den Fall umfassen soll, dass das wässrige Medium einen Gehalt bzw. einen Anteil des chemischen Stoffs der Anlage aufweist, als auch den Fall, dass das wässrige Medium keinen Gehalt bzw. keinen Anteil des chemischen Stoffs der Anlage aufweist, was erfindungsgemäß sensorisch zu bestimmen und auszuwerten ist, wie nachfolgend noch näher beschrieben werden wird.

In jedem Fall weist das erfindungsgemäße Flüssigkeitsrückhaltesystem einen betätigbaren Auslass, vorzugsweise ein Ventil, auf, welcher ausgebildet ist, ein Abfließen des den chemischen Stoff der Anlage enthaltenden wässrigen Mediums aus der Auffangwanne zuzulassen oder zu unterbinden. Somit kann der betätigbare Auslass wenigstens zwei Stellungen aufweisen bzw. einnehmen, um entweder geöffnet zu sein und ein Ausfließen bzw. ein Ausströmen des den chemischen Stoff der Anlage enthaltenden wässrigen Mediums zuzulassen bzw. zu ermöglichen, oder um geschlossen zu sein und ein Ausfließen bzw. ein Ausströmen des den chemischen Stoff der Anlage enthaltenden wässrigen Mediums zu unterbinden bzw. zu verhindern. Ggfs. kann das Maß des Öffnens des betätigbaren Auslasses veränderbar bzw. einstellbar sein, um die Menge des ausfließenden bzw. ausströmenden ein Ausfließen bzw. ein Ausströmen des den chemischen Stoff der Anlage enthaltenden wässrigen Mediums zu beeinflussen bzw. zu regulieren. Diese Stellungen können ebenfalls als offen bzw. geöffnet angesehen werden, lediglich in einem unterschiedlichen Maße.

In jedem Fall ist das Flüssigkeitsrückhaltesystem und vorzugsweise eine Steuerungseinheit des Flüssigkeitsrückhaltesystem oder eine ein Schwingungselement aufweisende Sensoreinheit des Flüssigkeitsrückhaltesystem ausgebildet und eingerichtet:
- ein Schwingungselement, vorzugsweise eine Schwinggabel, welches in Kontakt mit dem den chemischen Stoff der Anlage enthaltenden wässrigen Medium steht, mit seiner Resonanzfrequenz anzuregen,
- eine Resonanzfrequenz des Schwingungselements zu erfassen,
- den Gehalt des chemischen Stoffes der Anlage im wässrigen Medium basierend auf der erfassten Resonanzfrequenz des Schwingungselements zu bestimmen und
- den betätigbaren Auslass in Abhängigkeit des bestimmten Gehalts des chemischen Stoffes der Anlage im wässrigen Medium zu öffnen oder zu schließen.

Der vorliegenden Erfindung geht von der Erkenntnis aus, dass, wie eingangs beschrieben, eine Auffangwanne eines Flüssigkeitsrückhaltesystems nicht nur den eigentlichen chemischen Stoff der entsprechenden Anlage, welcher von der Umwelt ferngehalten und daher aufgefangen werden soll, aufnehmen kann, sondern aufgrund der Konstruktion der Auffangwanne, welche üblicherweise nach oben hin offen ist, üblicherweise deutlich mehr andere Flüssigkeiten wie insbesondere Wasser, insbesondere in Form von Regenwasser und bzw. oder Kondenswasser, von der Auffangwanne aufgenommen werden als der chemische Stoff der Anlage. Dies kann zu einem ungewollten bzw. unkontrollierten Überlaufen der Auffangwanne führen, wodurch dann doch der chemische Stoff der Anlage, falls dieser im wässrigen Medium vermischt bzw. gelöst vorhanden ist, in die Umwelt gelangen könnte, was eigentlich durch die Auffangwanne des Flüssigkeitsrückhaltesystems verhindert werden soll.

Um ein derartiges Überlaufen der Auffangwanne zu verhindern, ist es bekannt, das wässrige Medium aus der Auffangwanne grundsätzlich kontrolliert ablaufen zu lassen, insbesondere in eine Kanalisation. Dies kann jedoch nur dann erfolgen, falls der chemische Stoff der Anlage nicht bzw. in einem zulässig geringen Maße, insbesondere mit einem Gehalt bzw. Anteil im wässrigen Medium unterhalb eines Grenzwertes bzw. bis zum Erreichen eines Grenzwertes, was insbesondere gesetzlich vorgegeben sein kann, im wässrigen Medium enthalten ist. Ist dieses Maß unzulässig hoch und wird insbesondere der Grenzwert des Gehalts bzw. des Anteils im wässrigen Medium erreicht bzw. überschritten, so würde das weitere Ablaufen des den chemischen Stoff der Anlage enthaltenden wässrigen Medium zu genau derjenigen Umweltbeeinflussung bzw. Umweltverschmutzung führen, welche gerade durch die Verwendung der Auffangwanne vermieden werden soll.

Entsprechend wird der Gehalt des chemischen Stoffes der Anlage im wässrigen Medium bestimmt und in Abhängigkeit des bestimmten Gehalts des chemischen Stoffes der Anlage im wässrigen Medium der betätigbare Auslass geöffnet oder geschlossen. Somit kann das den chemischen Stoff der Anlage enthaltende wässrige Medium aus der Auffangwanne abgelassen werden, falls gar kein bzw. ein ausreichend geringer Gehalt des Stoffes der Anlage im wässrigen Medium enthalten ist. Wird hingegen ein zu hoher Gehalt des Stoffes der Anlage im wässrigen Medium erkannt, so kann der betätigbare Auslass geschlossen und somit ein Austreten des den chemischen Stoff der Anlage in einem zu hohen Maße enthaltenden wässrigen Mediums verhindert werden. Das den chemischen Stoff der Anlage enthaltende wässrige Medium kann dann anders entnommen bzw. entsorgt werden.

Erfindungsgemäß wird der Gehalt des chemischen Stoffes der Anlage im wässrigen Medium bestimmt, indem die Resonanzfrequenz eines Schwingungselements sensorisch erfasst und der Gehalt des chemischen Stoffes der Anlage im wässrigen Medium basierend auf der erfassten Resonanzfrequenz des Schwingungselements bestimmt wird. Das Schwingungselement kann ein schwingfähiges sensorisches System in Form einer Schwinggabel sein, welches mit seiner Resonanzfrequenz angeregt werden kann. Dies kann mittels einer entsprechenden geeigneten Sensorik erfolgen, welche hierzu mit dem den chemischen Stoff der Anlage enthaltenden wässrigen Medium in Kontakt stehen kann, um die Resonanzschwingungen des Schwingungselements zu erzeugen sowie zu erfassen und die erfassten Resonanzschwingungen des Schwingungselements entsprechend auszuwerten.

Dabei liegt der vorliegenden Erfindung die Erkenntnis zugrunde, dass die Resonanzfrequenz des schwingfähigen sensorischen Systems durch den Gehalt des sich im wässrigen Mediums befindlichen chemischen Stoffes der Anlage, welcher bzw. dessen Gehalt zu detektieren ist, bei Kontakt des wässrigen Mediums mit dem schwingungsfähigen sensorischen System derart beeinflusst werden kann, dass anhand der aktuellen Resonanzfrequenz des schwingungsfähigen sensorischen Systems ein ausreichender Rückschluss auf den Gehalt des chemischen Stoffes der Anlage im wässrigen Medium gezogen werden kann. Entsprechend kann diese Art der Messung verwendet werden, um den Gehalt des chemischen Stoffes der Anlage im wässrigen Medium zumindest derart ausreichend zu bestimmen, um in Reaktion auf den derart bestimmten Gehalt des chemischen Stoffes der Anlage im wässrigen Medium wenigstens den betätigbaren Auslass zu öffnen oder zu schließen.

Vorteilhaft ist bei einer Bestimmung des Gehalts des chemischen Stoffes der Anlage im wässrigen Medium basierend auf erfassten Resonanzfrequenz des sensorischen Systems, dass die Nachteile der bisher bekannten Messverfahren für derartige Anwendungen vermieden oder zumindest reduziert werden können. So haben grobe Verschmutzungen wie beispielsweise Partikel, Schwebestoffe und dergleichen im wässrigen Medium sowie im wässrigen Medium gelöste Salze einen vergleichsweise geringen bis gar keinen relevanten Einfluss auf die Resonanzfrequenz des Schwingungselements bzw. auf die Dichte des den chemischen Stoff der Anlage enthaltenden wässrigen Mediums, so dass diese Einflüsse ausgeschlossen bzw. vernachlässigt werden können. Auch kann das Erfassen der Resonanzfrequenz des Schwingungselements auch dann erfolgen, wenn zuvor kein wässriges Medium vorhanden war. Ferner kann das Erfassen der Resonanzfrequenz des Schwingungselements sowohl bei stehenden als auch bei fließenden bzw. bei strömenden wässrigen Medien erfolgen.

Die entsprechenden Tätigkeiten des Erfassens, des Bestimmens sowie des Schließens oder Öffnens, welche von Komponenten des Flüssigkeitsrückhaltesystems ausgeführt werden können, können vorzugsweise von einer Steuerungseinheit des Flüssigkeitsrückhaltesystems oder von einer das Schwingungselements aufweisenden Sensoreinheit des Flüssigkeitsrückhaltesystems ausgeführt oder veranlasst werden. Diese Tätigkeiten können auch als Verfahrensschritte angesehen bzw. durchgeführt werden. Hierdurch kann eine Koordinierung dieser Tätigkeiten seitens der Steuerungseinheit erfolgen, welche zusätzlich noch weitere Tätigkeiten übernehmen bzw. ausführen kann, wie nachfolgend noch näher beschrieben werden wird.

Mit anderen Worten liegt der vorliegenden Erfindung die Erkenntnis zugrunde, dass ein Schwingungselement als schwingfähiges sensorisches System, insbesondere als Schwinggabel, mit ihrer Resonanzfrequenz angeregt werden kann. Die Resonanzfrequenz des Schwingungselements ist u.a. abhängig von der Geometrie und der Masse des Schwingungselements. Ferner ist die Resonanzfrequenz des Schwingungselements von der Dichte des Mediums, welches das Schwingungselements umgibt bzw. mit welchem das Schwingungselement in Kontakt steht, abhängig.

Somit kann das Schwingungselement ausgebildet und eingerichtet sein, sowohl in Schwingungen versetzt zu werden als auch die resultierende Schwingung zu erfassen, d.h. aufzunehmen. Dies kann insbesondere dadurch umgesetzt werden, indem das Schwingungselement bzw. die Schwinggabel zwei Piezokeramiken enthält, von denen eine Piezokeramik zum Schwingen angeregt bzw. in Schwingungen versetzt und die andere Piezokeramik die resultierende Schwingung erfassen bzw. aufnehmen kann. Das Auffinden der Resonanzfrequenz des Schwingungselements kann über eine Phasen- oder Peak-Detektionsschaltung erfolgen, indem so lange die Frequenz von einem bestimmten Ausgangswert erhöht oder verringert wird, bis die Resonanzfrequenz des Schwingungselements gefunden wurde, was daran erkannt werden kann, dass die höchste Schwingungsamplitude auftritt und bzw. oder eine gewisse Phasenverschiebung zwischen der Schwingungs-Piezokeramik bzw. der Anregungs-Piezokeramik und der Aufnahme-Piezokeramik bzw. der Sensor-Piezokeramik vorliegt.

Dieses Messprinzip beruht darauf, dass das Schwingungselement in eine newtonsche Flüssigkeit als wässriges Medium eingetaucht werden kann, wodurch die Masse des wässrigen Mediums die Schwingung des Schwingungselements je nach Dichte des wässrigen Mediums mehr oder weniger dämpft. Diese Dämpfung der Schwingung des Schwingungselements durch das wässrige Medium bewirkt eine Änderung der Resonanzfrequenz des Schwingungselements. Nun kann, wie zuvor beschrieben, nach der aktuellen Resonanzfrequenz des Schwingungselements gesucht bzw. diese detektiert werden, welche somit von der Dichte des wässrigen Mediums abhängt, in welchem das Schwingungselement schwingt. Somit kann basierend auf der sensorisch erfassten aktuellen Resonanzfrequenz des Schwingungselements schlussendlich die Dichte des das Schwingungselement umgebenden Mediums berechnet bzw. bestimmt werden.

Gelangt nun ein zu erkennender bzw. zu detektierender chemischer Stoff wie beispielsweise Glykol ins wässrige Medium bzw. ins Wasser, ändert sich die Dichte und damit die Masse pro Volumen des wässrigen Mediums in Abhängigkeit der Menge des chemischen Stoffes. Diese veränderte Dichte des wässrigen Mediums verändert wiederum die Resonanzfrequenz des Schwingungselements entsprechend. Über diese geänderte Resonanzfrequenz des Schwingungselements kann auf die geänderte bzw. aktuelle Dichte des wässrigen Mediums rückgeschlossen werden. Ist nun die Dichte von beispielsweise Wasser und Glykol bekannt, kann anhand der gemessenen bzw. erfassten geänderten Dichte aufgrund des ins Wasser hinzugefügten Glykols die Glykolkonzentration im Wasser berechnet bzw. bestimmt werden. Dies gilt vergleichbar für anderer chemischen Stoff von Anlagen sowie für andere wässrige Medien.

Gemäß einem Aspekt der Erfindung weist das Flüssigkeitsrückhaltesystem ferner einen Temperatursensor auf, welcher ausgebildet und eingereicht ist, die Temperatur des den chemischen Stoff der Anlage enthaltenden wässrigen Mediums und bzw. oder des Schwingungselements zu erfassen, wobei das Flüssigkeitsrückhaltesystem, vorzugsweise eine Steuerungseinheit des Flüssigkeitsrückhaltesystems oder von einer das Schwingungselements aufweisenden Sensoreinheit des Flüssigkeitsrückhaltesystems, ferner ausgebildet und eingerichtet ist, den Gehalt des chemischen Stoffes der Anlage im wässrigen Medium basierend auf der erfassten Resonanzfrequenz des Schwingungselements und auf der erfassten Temperatur des den chemischen Stoff der Anlage enthaltenden wässrigen Mediums und bzw. oder des Schwingungselements zu bestimmen.

Mittels wenigstens eines Temperatursensors bzw. jeweils eines Temperatursensors kann eine sensorische Erfassung der Temperatur des den chemischen Stoff der Anlagen enthaltenden wässrigen Mediums und bzw. oder des Schwingungselements derart erfolgen, dass die erfasste Temperatur bzw. die erfassten Temperaturen beim Bestimmen des Gehalts des chemischen Stoffes der Anlage im wässrigen Medium, vorzugsweise seitens der Steuerungseinheit, berücksichtigt werden kann bzw. können. Dies kann vorzugsweise unmittelbar dort erfolgen, wo ein entsprechender Sensor oder dergleichen die Resonanzfrequenz des Schwingungselements erfassten kann.

Diesem Aspekt der Erfindung liegt die Erkenntnis zugrunde, dass die Dichte des den chemischen Stoff der Anlage enthaltenden wässrigen Mediums und damit auch die Resonanzfrequenz des im wässrigen Medium eingetauchten Schwingungselements in einem gewissen Maße von der Temperatur des den chemischen Stoff der Anlagen enthaltenden wässrigen Mediums abhängt.

Dies gilt zum einen für das den chemischen Stoff der Anlage enthaltenden wässrigen Medium selbst bzw. direkt. Denn da beispielsweise Wasser und Glykol unterschiedliche Dichten bei unterschiedlichen Temperaturen aufweisen, kann die Berücksichtigung der sensorisch erfassten Temperatur des den chemischen Stoff der Anlage enthaltenden wässrigen Mediums die Genauigkeit des Bestimmens der Dichte erhöhen bzw. verbessern, d.h. die sensorisch erfasste Temperatur des den chemischen Stoff der Anlage enthaltenden wässrigen Mediums kann als Kompensation in die Dichteberechnung miteinbezogen werden.

Dies gilt zum anderen hinsichtlich der Temperatur des Schwingungselements. Wird beispielsweise eine Schwinggabel als Schwingungselement verwendet, so ist auch die Resonanzfrequenz der Schwinggabel temperaturabhängig, beispielsweise da sich die Gabelzinken bei Erwärmung ausdehnen und sich damit die Geometrie der Schwinggabel ändert. Entsprechend kann es durch die Kenntnis der sensorisch erfassten Temperatur der Schwinggabel ermöglicht werden, die Temperatur der Schwingkabel beim Bestimmen der Dichte des den chemischen Stoff der Anlage enthaltenden wässrigen Mediums zu berücksichtigen, d.h. die Temperatur der Schwinggabel kann als Kompensation in die Dichteberechnung miteinbezogen werden.

Somit kann die Genauigkeit des Bestimmens des Gehalts des chemischen Stoffes der Anlage im wässrigen Medium basierend auf der erfassten Resonanzfrequenz des Schwingungselements erhöht werden, indem zusätzlich die Temperatur des den chemischen Stoff der Anlage enthaltenden wässrigen Mediums und bzw. oder des Schwingungselements erfasst, bestimmt und hierbei berücksichtigt werden kann. Dies kann auch als Kompensation von temperaturabhängigen Effekten bzw. Einflüssen auf das Bestimmen des Gehalts des chemischen Stoffes der Anlage im wässrigen Medium basierend auf der erfassten Resonanzfrequenz des Schwingungselements angesehen werden.

Gemäß einem weiteren Aspekt der Erfindung ist das Flüssigkeitsrückhaltesystem, vorzugsweise eine Steuerungseinheit des Flüssigkeitsrückhaltesystems oder eine das Schwingungselement aufweisende Sensoreinheit des Flüssigkeitsrückhaltesystems, ausgebildet und eingerichtet,
- die absolute Dichte des den chemischen Stoff der Anlage enthaltenden wässrigen Mediums basierend auf der erfassten Resonanzfrequenz des Schwingungselements, vorzugsweise und auf der erfassten Temperatur des den chemischen Stoff der Anlage enthaltenden wässrigen Mediums und bzw. oder des Schwingungselements, zu bestimmen und
- den Gehalt des chemischen Stoffes der Anlage des den chemischen Stoff der Anlage enthaltenden wässrigen Mediums basierend auf der bestimmten absoluten Dichte des den chemischen Stoff der Anlage enthaltenden wässrigen Mediums zu bestimmen.

Dies kann eine konkrete Möglichkeit der Umsetzung darstellen, indem zunächst die absolute Dichte des den chemischen Stoff der Anlage enthaltenden wässrigen Mediums basierend auf der erfassten Resonanzfrequenz des Schwingungselements, wie zuvor beschrieben, bestimmt wird. Wie bereits erwähnt, fällt je nach Dichte des das Schwingungselement umgebenden Mediums die Resonanzfrequenz des Schwingungselements unterschiedlich aus. Bei Kenntnis der Dichte des wässrigen Mediums wie beispielsweise Wasser in Form von Regenwasser und bzw. oder von Kondenswasser und bei Kenntnis der Dichte des sich in der Anlage befindlichen chemischen Stoffes wie beispielsweise eines Gefrierschutzmittels, insbesondere Glykol, welcher detektiert werden soll, kann über die gemessene Dichte auf die Konzentration des evtl. aus der Anlage ausgetretenen chemischen Stoffes rückgeschlossen werden.

Auch hierzu kann vorzugsweise zusätzlich dessen erfasste Temperatur des wässrigen Mediums und bzw. oder des Schwingungselements verwendet werden, wie zuvor bereits beschrieben, um die Genauigkeit des Bestimmens der absolute Dichte des den chemischen Stoff der Anlage enthaltenden wässrigen Mediums zu erhöhen bzw. entsprechend temperaturabhängige Einflüsse zu kompensieren. In jedem Fall kann dann der Gehalt des chemischen Stoffes der Anlage im den chemischen Stoff der Anlage enthaltenden wässrigen Medium basierend auf der bestimmten absoluten Dichte des den chemischen Stoff der Anlage enthaltenden wässrigen Mediums bestimmt werden.

Gemäß einem weiteren Aspekt der Erfindung weist das Flüssigkeitsrückhaltesystem, vorzugsweise eine Steuerungseinheit des Flüssigkeitsrückhaltesystems oder eine das Schwingungselement aufweisende Sensoreinheit des Flüssigkeitsrückhaltesystems, einen vorbestimmten Grenzwert des bestimmten Gehalts des chemischen Stoffes der Anlage im wässrigen Medium auf, wobei das Flüssigkeitsrückhaltesystem, vorzugsweise eine Steuerungseinheit des Flüssigkeitsrückhaltesystems oder eine das Schwingungselement aufweisende Sensoreinheit des Flüssigkeitsrückhaltesystems, ausgebildet und eingerichtet ist,
- den betätigbaren Auslass solange zu öffnen, wie der bestimmte Gehalt des chemischen Stoffes der Anlage im wässrigen Medium unterhalb des vorbestimmten Grenzwerts liegt, und
- den betätigbaren Auslass zu schließen, sobald der bestimmte Gehalt des chemischen Stoffes der Anlage im wässrigen Medium den vorbestimmten Grenzwert erreicht,
   oder
- den betätigbaren Auslass solange zu öffnen, bis der bestimmte Gehalt des chemischen Stoffes der Anlage im wässrigen Medium den vorbestimmten Grenzwert erreicht, und
- den betätigbaren Auslass zu schließen, sobald der bestimmte Gehalt des chemischen Stoffes der Anlage im wässrigen Medium oberhalb des vorbestimmten Grenzwerts liegt.

Dies können konkrete Möglichkeiten darstellen, um anhand des bestimmten Gehalts des chemischen Stoffes der Anlage im wässrigen Medium zu unterscheiden, ob der betätigbare Auslass zu öffnen oder zu schließen ist.

Gemäß einem weiteren Aspekt der Erfindung weist das Schwingungselement eine hydrophil ausgebildete Oberfläche, vorzugsweise mit einem Kontaktwinkel zwischen der Oberfläche und dem den chemischen Stoff der Anlage enthaltenden wässrigen Medium von weniger als 30°, auf. Dies kann durch Auftragen einer Polymer- oder Silikat-basierten Beschichtung umgesetzt werden, welche eine hydrophile Oberfläche aufweist. Hydrophil ausgebildete Oberflächen lassen sich aber auch durch Laserbearbeitung erzeugen.

Auf diese Art und Weise kann ein Anhaften von aus dem den chemischen Stoff der Anlage enthaltenden wässrigen Medium ausgasenden Luftblasen an der Oberfläche des Schwingungselements verhindert oder zumindest reduziert werden. Dies kann die Genauigkeit der erfassten Schwingungsfrequenz bzw. Resonanzfrequenz mittels des Schwingungselements verbessern, da Luftblasen, welche an der Oberfläche des Schwingungselements anhaften, das Messergebnis verfälschen können, sodass die hierauf aufbauenden Tätigkeiten bzw. Schritte, um den Gehalt des chemischen Stoffes der Anlage im wässrigen Medium basierend auf der erfassten Resonanzfrequenz des Schwingungselements zu bestimmen, in ihrer Genauigkeit reduziert bzw. negativ beeinflusst werden können. Die Verhinderung bzw. die Reduzierung der Anhaftung von Luftblasen an der Schwinggabeloberfläche kann dabei besonders wirkungsvoll und gleichzeitig mit möglichst geringem Aufwand durch eine hydrophilisierte Oberfläche mit einem Kontaktwinkel zwischen Oberfläche und wässrigem Medium von weniger als 30° erreicht werden. Durch die starke Benetzbarkeit des wässrigen Mediums mit der Oberfläche durch Ausgasung können entstehende Luftblasen daran gehindert werden, sich an der Oberfläche des Schwingungselements anzuhaften.

Gemäß einem weiteren Aspekt der Erfindung weist das Flüssigkeitsrückhaltesystem ferner einen Abfluss auf, welcher die Auffangwanne mit dem betätigbaren Auslass verbindet, wobei das Schwingungselement, vorzugsweise eine das Schwingungselement aufweisende Sensoreinheit des Flüssigkeitsrückhaltesystems, im Bereich des Abflusses, vorzugsweise im Bereich einer Senke des Abflusses, angeordnet ist.

Dies kann dahingehend vorteilhaft sein, um die sensorische Erfassung der Resonanzfrequenz des Schwingungselements genau dort durchzuführen, wo das den chemischen Stoff der Anlage enthaltende wässrige Medium aus der Auffangwanne abgelassen werden kann und somit der chemische Stoff in unzulässig hoher Konzentration in die Umwelt gelangen könnte. Dies kann insbesondere auch bei geringen Mengen des den chemischen Stoff der Anlage enthaltenden wässrigen Mediums sichergestellt werden, indem sich das wässrige Medium in einer Senke bzw. in einem Siphon sammeln und genau dort die sensorische Erfassung der Resonanzfrequenz des Schwingungselements und der hierauf basierenden Bestimmung des Gehalts chemischen Stoffes im den chemischen Stoff der Anlage enthaltenden wässrigen Medium erfolgen kann.

Gemäß einem weiteren Aspekt der Erfindung weist das Flüssigkeitsrückhaltesystem ferner einen Abscheider auf, welcher vor dem Schwingungselement angeordnet ist.

Auf diese Art und Weisen können grobe Verschmutzungen wie beispielsweise Partikel, Schwebestoffe und dergleichen aus dem wässrigen Medium entfernt werden, so dass diese das Erfassen der Resonanzfrequenz des Schwingungselements nicht beeinflussen können, wenngleich das Messprinzip des Erfassens der Resonanzfrequenz des Schwingungselements bereits an sich vergleichsweise unempfindlich gegenüber groben Verschmutzungen sein kann. Dennoch können mögliche störende Einflüsse durch groben Verschmutzungen dadurch weiter reduziert bzw. ausgeschlossen werden, indem mittels eines Abscheiders derartige Verschmutzungen aus dem den chemischen Stoff der Anlage enthaltenden wässrigen Medium entfernt bzw. deren Maß reduziert werden kann.

Gemäß einem weiteren Aspekt der Erfindung ist das Flüssigkeitsrückhaltesystem, vorzugsweise mittels einer Steuerungseinheit oder mittels eines ein Schwingungselement aufweisende Sensoreinheit, ausgebildet und eingerichtet, eine Information über den bestimmten Gehalt des chemischen Stoffes der Anlage im wässrigen Medium und bzw. oder eine auf dem bestimmten Gehalt des chemischen Stoffes der Anlage im wässrigen Medium basierende Information, insbesondere eine Warnung, auszugeben und bzw. oder auszusenden.

Somit können diese Informationen, einzeln oder jeweils bzw. zusammen, nach außerhalb des Flüssigkeitsrückhaltesystems ausgegeben, was optisch und bzw. oder akustisch erfolgen kann, bzw. ausgesendet werden, was drahtgebunden oder drahtlos erfolgen kann. Auf diese Art und Weise können derartige Informationen außerhalb des Flüssigkeitsrückhaltesystems, insbesondere seitens einer Steuerungseinheit der Anlage selbst oder seitens einer Steuerungseinheit einer der Anlage übergeordneten technischen Einrichtung, erhalten bzw. empfangen und dort genutzt werden. Dies kann der Überwachung sowie der Reinigung des Flüssigkeitsrückhaltesystems dienen, falls der Anteil bzw. der Gehalt des chemischen Stoffes der Anlage im wässrigen Medium zu hoch ist und der betätigbare Auslass geschlossen wird bzw. wurde, so dass das den chemischen Stoff der Anlage in einem unzulässig hohen Maße enthaltende wässrige Medium anderweitig aus der Auffangwanne zu entfernen und zu entsorgen ist.

Gemäß einem weiteren Aspekt der Erfindung ist das Flüssigkeitsrückhaltesystem, vorzugsweise mittels einer Steuerungseinheit oder mittels eines ein Schwingungselement aufweisende Sensoreinheit, ausgebildet und eingerichtet, eine Information auszugeben und bzw. oder auszusenden, falls der betätigbare Auslass geschlossen wird bzw. ist und bzw. oder falls ein vorbestimmter Grenzwert des bestimmten Gehalts des chemischen Stoffes der Anlage im den chemischen Stoff der Anlage enthaltenden wässrigen Medium erreicht oder überschritten wird bzw. ist.

Dies können konkrete Möglichkeiten darstellen, im konkreten Fall eines überschrittenen vorbestimmten Grenzwertes des bestimmten Gehalts des chemischen Stoffes der Anlage im den chemischen Stoff der Anlage enthaltenden wässrigen Medium zu reagieren, wie zuvor bereits teilweise beschrieben.

Gemäß einem weiteren Aspekt der Erfindung ist das Flüssigkeitsrückhaltesystem, vorzugsweise eine Steuerungseinheit des Flüssigkeitsrückhaltesystems oder eine das Schwingungselement aufweisende Sensoreinheit des Flüssigkeitsrückhaltesystems, ausgebildet und eingerichtet ist, das Erfassen der Resonanzfrequenz des Schwingungselements und das Bestimmen des Gehalts des chemischen Stoffes der Anlage im wässrigen Medium basierend auf der erfassten Resonanzfrequenz des Schwingungselements in vorbestimmten regelmäßigen zeitlichen Abständen auszuführen.

Dies kann die Umsetzung des Bestimmens des Gehalts des chemischen Stoffes der Anlage im wässrigen Medium in intermittierenden Messzyklen ermöglichen, d.h. dies kann nicht kontinuierlich, sondern in regelmäßigen oder unregelmäßigen Abständen mit Unterbrechungen durchgeführt werden. Dies kann einerseits ausreichend häufig erfolgen, um auf einen unzulässig hohen Gehalt des chemischen Stoffes der Anlage im wässrigen Medium reagieren zu können, jedoch andererseits den Aufwand der Durchführung dieses Vorgangs vergleichsweise geringhalten. Die intermittierende Messung kann den Langzeitdrift des Systems verringern und somit die Wartungsintervalle verlängern.

Gemäß einem weiteren Aspekt der Erfindung ist der chemische Stoff ein Gefrierschutzmittel, vorzugsweise Glykol, die Anlage ist einer Kälte- oder Klimaanlage und das den chemischen Stoff der Anlage enthaltende wässrige Medium ist Wasser, vorzugsweise Kondenswasser und bzw. oder Regenwasser.

Dies kann eine konkrete Möglichkeit der Umsetzung darstellen, wie zuvor bereits beschrieben.

Die Erfindung betrifft auch ein System mit einer Anlage, vorzugsweise einer Kälte- oder Klimaanlage, welche einen chemischen Stoff, vorzugsweise ein Gefrierschutzmittel, besonders vorzugsweise Glykol, enthält, und mit einem Flüssigkeitsrückhaltesystem wie zuvor beschrieben, wobei die Anlage zumindest abschnittsweise entlang der vertikalen Achse oberhalb der Auffangwanne des Flüssigkeitsrückhaltesystem angeordnet ist.

Auf diese Art und Weise kann das erfindungsgemäße Flüssigkeitsrückhaltesystem bei einer entsprechenden Anlage angewendet bzw. eingesetzt werden, um die Eigenschaften und Vorteile des erfindungsgemäßen Flüssigkeitsrückhaltesystems dort umzusetzen und zu nutzen.

Ein Ausführungsbeispiel und weitere Vorteile der Erfindung werden nachstehend im Zusammenhang mit den folgenden Figuren rein schematisch dargestellt und näher erläutert. Darin zeigt:
- Figur 1: eine Darstellung eines erfindungsgemäßen Flüssigkeitsrückhaltesystems mit Anlage; und
- Figur 2: eine Detailansicht eines Abflusses des Flüssigkeitsrückhaltesystems der Figur 1.

Die o.g. Figuren werden in kartesischen Koordinaten betrachtet. Es erstreckt sich eine Längsachse X, welche auch als Tiefe X oder als Länge X bezeichnet werden kann. Senkrecht zur Längsachse X erstreckt sich eine Querachse (nicht dargestellt), welche auch als Breite bezeichnet werden kann. Senkrecht sowohl zur Längsachse X als auch zur Querachse erstreckt sich eine vertikale Achse Z, welche auch als Höhe Z bezeichnet werden kann und der Richtung der Schwerkraft bzw. der Gravitationskraft entspricht. Die Längsachse X und die Querachse bilden gemeinsam die Horizontale, welche auch als horizontale Ebene bezeichnet werden kann.

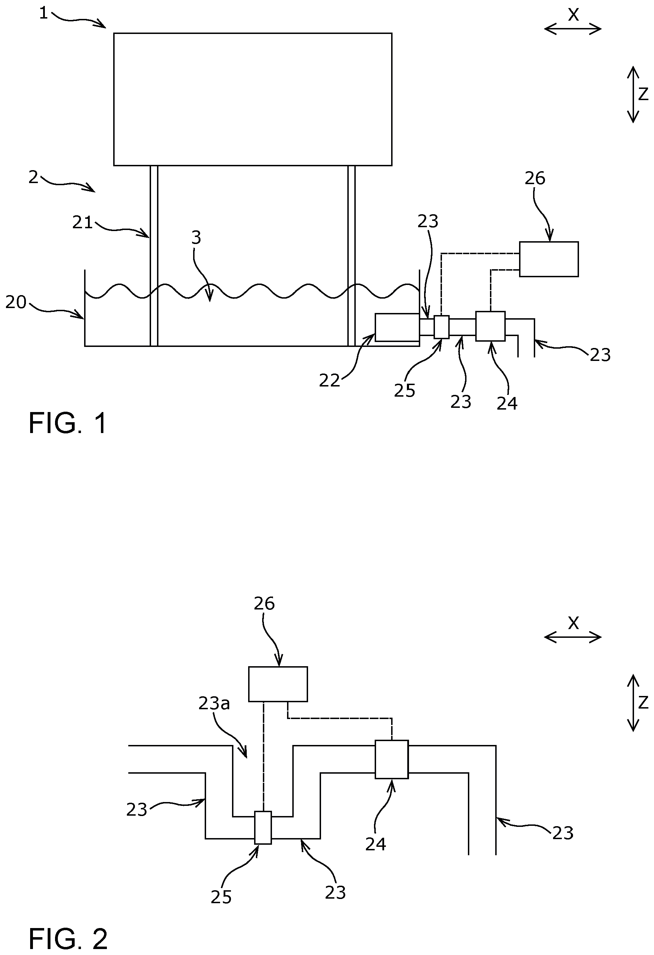

Figur 1 zeigt eine Darstellung eines erfindungsgemäßen Flüssigkeitsrückhaltesystems 2 mit Anlage 1.

Figur 2 zeigt eine Detailansicht eines Abflusses 23 des Flüssigkeitsrückhaltesystems 2 der Figur 1.

Es wird eine Anlage 1 in Form einer Kälte- oder Klimaanlage 1 betrachtet. Die Kälte- oder Klimaanlage 1 verwendet zum Betrieb einen chemischen Stoff in Form von Glykol als flüssiges Gefrierschutzmittel.

Um ungewollt aus der Kälte- oder Klimaanlage 1 austretendes Glykol von der Umwelt bzw. von der Umgebung und insbesondere vom Untergrund bzw. vom Grundwasser fernzuhalten, wird ein erfindungsgemäßes Flüssigkeitsrückhaltesystem 2 verwendet. Das Flüssigkeitsrückhaltesystem 2 weist eine Auffangwanne 20 auf, welche auch als Auffangbecken 20 bezeichnet werden kann. Mittels eines Gestells 21 ist die Kälte- oder Klimaanlage 1 entlang der vertikalen Achse Z oberhalb der Auffangwanne 20 des Flüssigkeitsrückhaltesystems 2 aufgeständert, so dass austretendes Glykol nach unten abtropfen oder am Gestell 21 herunterlaufen und jeweils von der Auffangwanne 20 aufgefangen werden kann.

Da die Auffangwanne 20 entsprechend nach oben hin offen ausgebildet ist und die betrachtete Kälte- oder Klimaanlage 1 im Freien aufgestellt ist, kann auch Regenwasser 3 in die Auffangwanne 20 gelangen. Ebenso kann sich an der Kälte- oder Klimaanlage 1 Kondenswasser 3 bilden, welche ebenfalls, d.h. vergleichbar dem Glykol, nach unten abtropfen oder am Gestell 21 herunterlaufen und so in die Auffangwanne 20 gelangen kann. Entsprechend kann sich in der Auffangwanne 20 ein Gemisch von Regenwasser 3 und bzw. oder Kondenswasser 3 bilden, welche als Wasser 3 mit dem Glykol gemischt sind. Dieses Gemisch kann als Glykol-enthaltendes wässriges Medium 3 bzw. als glykolhaltiges wässriges Medium 3 bezeichnet werden.

Das glykolhaltige wässrige Medium 3 kann über einen Abfluss 23, welcher auch als Wannenablauf 23 bezeichnet werden kann, aus der Auffangwanne 20 in eine Kanalisation (nicht dargestellt) ablaufen bzw. abgeführt werden. Damit jedoch lediglich dann das glykolhaltige wässrige Medium 3 aus der Auffangwanne 20 über den Abfluss 23 gelangen kann, wenn der Glykolgehalt ausreichend niedrig ist, um für das Abwasser der Kanalisation bzw. für die Umwelt unbedenklich zu sein, ist ein betätigbarer Auslass 24 in Form eines Ventils 24 bzw. eines Absperrventils 24 vorgesehen, welches mittels eines Antriebs bzw. mittels eines Aktuators (nicht dargestellt) geöffnet und geschlossen werden kann, um das Abfließen des glykolhaltigen wässrigen Mediums 3 aus der Auffangwanne 20 über den Abfluss 23 zu ermöglichen oder zu unterbinden.

Um diese Funktion im Rahmen eines In-Line Messverfahrens durchführen zu können, ist im Bereich des Ablaufs 23, siehe Figur 1, und dort insbesondere in einer Senke 23a bzw. in einem Siphon 23a des Abflusses 23 eine Sensoreinheit 25 vorgesehen. Die Sensoreinheit 25 weist ein Schwingungselement in Form einer Schwinggabel auf, welche in Kontakt mit dem glykolhaltigen wässrigen Medium 3 steht. Dabei ist die Schwinggabel ausgebildet, mit seiner Resonanzfrequenz angeregt zu werden und somit im glykolhaltigen wässrigen Medium 3 eingetaucht auf seiner eigenen Resonanzfrequenz zu schwingen. Die Schwinggabel kann eine eigene Resonanzfrequenz erfassen. Dies kann jeweils über Piezoelemente der Schwinggabel erfolgen. Die Sensoreinheit 25 weist ferner einen integrierten Temperatursensor (nicht dargestellt) auf, um die Temperatur des glykolhaltigen wässrigen Mediums 3 sowie der Schwinggabel zu erfassen.

Im Betrieb der Kälte- oder Klimaanlage 1 sowie des Flüssigkeitsrückhaltesystems 2 wird seitens der Sensoreinheit 25 die Resonanzfrequenz der Schwinggabel erfasst. Die Sensoreinheit 25 bestimmt dann die absolute Dichte des glykolhaltigen wässrigen Mediums 3 basierend auf der erfassten Resonanzfrequenz der Schwinggabel und auf der erfassten Temperatur des glykolhaltigen wässrigen Mediums 3 und des Schwingungselements. Dann wird seitens der Sensoreinheit 25 der Gehalt des Glykols im glykolhaltigen wässrigen Mediums 3 basierend auf der bestimmten absoluten Dichte des glykolhaltigen wässrigen Mediums 3 bestimmt. Der bestimmte Gehalt des Glykols im glykolhaltigen wässrigen Mediums 3 wird nun von der Sensoreinheit 25 an eine Steuerungseinheit 26 des Flüssigkeitsrückhaltesystems 2 bzw. an eine Anlagensteuerung 26 übertragen.

Die Steuerungseinheit 26 vergleicht intermittierend den jeweils von der Sensoreinheit 25 aktuell erhaltenen Gehalt des Glykols im glykolhaltigen wässrigen Mediums 3 mit einem vorbestimmten Grenzwert des Gehalts des Glykols im glykolhaltigen wässrigen Mediums 3, welche vom Betreiber der Kälte- oder Klimaanlage 1 vorgegeben und in der Steuerungseinheit 26 hinterlegt sein kann. Da die Steuerungseinheit 26 das Ventil 24 steuern kann, wird das Ventil 24 solange geöffnet bzw. offengelassen, wie der bestimmte Gehalt des Glykols im glykolhaltigen wässrigen Mediums 3 unterhalb des vorbestimmten Grenzwerts liegt. In geöffneten Zustand des Ventils 24 kann somit das glykolhaltige wässrige Medium 3 mittels des Auslasses 23 in die Kanalisation abgeführt werden.

Sobald der bestimmte Gehalt des Glykols im glykolhaltigen wässrigen Mediums 3 den vorbestimmten Grenzwert erreicht, betätigt die Steuerungseinheit 26 das Ventil 24, um dieses zu schließen und so einen weiteren Austritt des glykolhaltigen wässrigen Mediums 3 mittels des Auslasses 23 in die Kanalisation zu unterbinden bzw. zu verhindern. Auf diese Art und Weise kann das wässrige Medium 3, sollte dessen Glykolgehalt zu hoch sein, von der Kanalisation ferngehalten werden.

Die Steuerungseinheit 26 kann die Information des bestimmten Gehalts des Glykols im glykolhaltigen wässrigen Mediums 3 zusätzlich nach außerhalb des Flüssigkeitsrückhaltesystems 2 aufgeben, beispielsweise an eine Steuerungseinheit der Kälte- oder Klimaanlage 1, um dort genutzt und bzw. oder angezeigt zu werden. Ferner kann die Steuerungseinheit 26 im Falle des Überschreitens des vorbestimmten Grenzwertes des Gehalts des Glykols im glykolhaltigen wässrigen Mediums 3 eine Warnung, z.B. an die Steuerungseinheit der Kälte- oder Klimaanlage 1 und bzw. oder an Personen, ausgeben bzw. aussenden, um hierauf aufmerksam zu machen.

Die Schwinggabel weist dabei eine hydrophil ausgebildete Oberfläche mit einem Kontaktwinkel zwischen der Oberfläche und dem glykolhaltigen wässrigen Medium 3 von weniger als 30° auf, um eine Entstehung bzw. Anhaftung von Luftblasen dort zu verhindern, welche das Erfassen der absoluten Dichte des der Schwinggabel umgebenden Mediums, und damit des Konzentrationsgehalt des chemischen Stoffs im Wasser, beeinträchtigen könnten. Hierzu weist die Oberfläche der Schwinggabel eine Polymer- oder Silikat-basierende Beschichtung auf. Das Flüssigkeitsrückhaltesystem 2 weist am Eingang des Abflusses 23 einen Abscheider 22 als Flüssigkeitsrückhaltevorrichtung 22 auf, um grobe Verschmutzungen von der Sensoreinheit 24 fernzuhalten.

Auf diese Art und Weise können die Möglichkeiten verbessert werden, einen unzulässig hohen Gehalt von Glykol im glykolhaltigen wässrigen Mediums 3 der Auffangwanne20 des Flüssigkeitsrückhaltesystems 2 der Kälte- oder Klimaanlage 1 zu erkennen und hierauf zu reagieren.

Insbesondere können durch die Verwendung einer Sensoreinheit 25, welche den Gehalt von Glykol im glykolhaltigen wässrigen Medium 3 basierend auf der bestimmten absoluten Dichte des glykolhaltigen wässrigen Mediums 3 sowie basierend auf der erfassten Temperatur des glykolhaltigen wässrigen Mediums 3 sowie der Schwinggabel bestimmen kann, die Nachteile der bisher bekannten Messverfahren vermieden oder zumindest reduziert werden.

### BEZUGSZEICHENLISTE (Teil der Beschreibung)

- X: Längsachse; Tiefe; Länge
- Z: vertikale Achse; Höhe

- 1: Anlage; Kälte- oder Klimaanlage

- 2: Flüssigkeitsrückhaltesystem
- 20: Auffangwanne; Auffangbecken
- 21: Gestell
- 22: Abscheider; Flüssigkeitsrückhaltevorrichtung
- 23: Abfluss; Wannenablauf
- 23a: Senke bzw. Siphon des Abflusses 23
- 24: betätigbarer Auslass; Ventil; Absperrventil
- 25: Sensoreinheit
- 26: Steuerungseinheit; Anlagensteuerung

- 3: wässriges Medium; Wasser; Kondenswasser und/oder Regenwasser

## Patentansprüche

1. Flüssigkeitsrückhaltesystem (2)
mit einer Auffangwanne (20), welche ausgebildet ist, einen chemischen Stoff einer Anlage (1) und ein wässriges Medium (3) gemeinsam aufzufangen, und
mit einem betätigbaren Auslass (24), vorzugsweise einem Ventil (24), welcher ausgebildet ist, ein Abfließen des den chemischen Stoff der Anlage (1) enthaltenden wässrigen Mediums (3) aus der Auffangwanne (20) zuzulassen oder zu unterbinden,
wobei das Flüssigkeitsrückhaltesystem (2), vorzugsweise eine Steuerungseinheit (26) des Flüssigkeitsrückhaltesystem (2) oder eine ein Schwingungselement aufweisende Sensoreinheit (25) des Flüssigkeitsrückhaltesystem (2), ausgebildet und eingerichtet ist:
• ein Schwingungselement, vorzugsweise eine Schwinggabel, welches in Kontakt mit dem den chemischen Stoff der Anlage (1) enthaltenden wässrigen Medium (3) steht, mit seiner Resonanzfrequenz anzuregen,
• eine Resonanzfrequenz des Schwingungselements zu erfassen,
• den Gehalt des chemischen Stoffes der Anlage (1) im wässrigen Medium (3) basierend auf der erfassten Resonanzfrequenz des Schwingungselements zu bestimmen und
• den betätigbaren Auslass (24) in Abhängigkeit des bestimmten Gehalts des chemischen Stoffes der Anlage (1) im wässrigen Medium (3) zu öffnen oder zu schließen.

2. Flüssigkeitsrückhaltesystem (2) nach Anspruch 1,
ferner mit einem Temperatursensor, welcher ausgebildet und eingereicht ist, die Temperatur des den chemischen Stoff der Anlage (1) enthaltenden wässrigen Mediums (3) und/oder des Schwingungselements zu erfassen,
wobei das Flüssigkeitsrückhaltesystem (2), vorzugsweise eine Steuerungseinheit (26) des Flüssigkeitsrückhaltesystem (2) oder eine das Schwingungselement aufweisende Sensoreinheit (25) des Flüssigkeitsrückhaltesystem (2), ferner ausgebildet und eingerichtet ist, den Gehalt des chemischen Stoffes der Anlage (1) im wässrigen Medium (3) basierend auf der erfassten Resonanzfrequenz des Schwingungselements und auf der erfassten Temperatur des den chemischen Stoff der Anlage (1) enthaltenden wässrigen Mediums (3) und/oder des Schwingungselements zu bestimmen.

3. Flüssigkeitsrückhaltesystem (2) nach Anspruch 1 oder 2,
wobei das Flüssigkeitsrückhaltesystem (2), vorzugsweise eine Steuerungseinheit (26) des Flüssigkeitsrückhaltesystem (2) oder eine das Schwingungselement aufweisende Sensoreinheit (25) des Flüssigkeitsrückhaltesystem (2), ausgebildet und eingerichtet ist,
• die absolute Dichte des den chemischen Stoff der Anlage (1) enthaltenden wässrigen Mediums (3) basierend auf der erfassten Resonanzfrequenz des Schwingungselements, vorzugsweise und auf der erfassten Temperatur des den chemischen Stoff der Anlage (1) enthaltenden wässrigen Mediums (3) und/oder des Schwingungselements, zu bestimmen und
• den Gehalt des chemischen Stoffes der Anlage (1) des den chemischen Stoff der Anlage (1) enthaltenden wässrigen Mediums (3) basierend auf der bestimmten absoluten Dichte des den chemischen Stoff der Anlage (1) enthaltenden wässrigen Mediums (3) zu bestimmen.

4. Flüssigkeitsrückhaltesystem (2) nach einem der vorangehenden Ansprüche,
wobei das Flüssigkeitsrückhaltesystem (2), vorzugsweise eine Steuerungseinheit (26) des Flüssigkeitsrückhaltesystem (2) oder eine das Schwingungselement aufweisende Sensoreinheit (25) des Flüssigkeitsrückhaltesystem (2), einen vorbestimmten Grenzwert des bestimmten Gehalts des chemischen Stoffes der Anlage (1) im wässrigen Medium (3) aufweist,
wobei das Flüssigkeitsrückhaltesystem (2), vorzugsweise eine Steuerungseinheit (26) des Flüssigkeitsrückhaltesystem (2) oder eine das Schwingungselement aufweisende Sensoreinheit (25) des Flüssigkeitsrückhaltesystem (2), ausgebildet und eingerichtet ist,
• den betätigbaren Auslass (24) solange zu öffnen, wie der bestimmte Gehalt des chemischen Stoffes der Anlage (1) im wässrigen Medium (3) unterhalb des vorbestimmten Grenzwerts liegt, und
• den betätigbaren Auslass (24) zu schließen, sobald der bestimmte Gehalt des chemischen Stoffes der Anlage (1) im wässrigen Medium (3) den vorbestimmten Grenzwert erreicht,
oder
• den betätigbaren Auslass (24) solange zu öffnen, bis der bestimmte Gehalt des chemischen Stoffes der Anlage (1) im wässrigen Medium (3) den vorbestimmten Grenzwert erreicht, und
• den betätigbaren Auslass (24) zu schließen, sobald der bestimmte Gehalt des chemischen Stoffes der Anlage (1) im wässrigen Medium (3) oberhalb des vorbestimmten Grenzwerts liegt.

5. Flüssigkeitsrückhaltesystem (2) nach einem der vorangehenden Ansprüche,
wobei das Schwingungselement eine hydrophil ausgebildete Oberfläche, vorzugsweise mit einem Kontaktwinkel zwischen der Oberfläche und dem den chemischen Stoff der Anlage (1) enthaltenden wässrigen Medium (3) von weniger als 30°, aufweist.

6. Flüssigkeitsrückhaltesystem (2) nach einem der vorangehenden Ansprüche,
ferner mit einem Abfluss (23), welcher die Auffangwanne (20) mit dem betätigbaren Auslass (24) verbindet,
wobei das Schwingungselement, vorzugsweise eine das Schwingungselement aufweisende Sensoreinheit (25) des Flüssigkeitsrückhaltesystems (2), im Bereich des Abflusses (23), vorzugsweise im Bereich einer Senke (23a) des Abflusses (23), angeordnet ist.

7. Flüssigkeitsrückhaltesystem (2) nach Anspruch 6,
ferner mit einem Abscheider (22), welcher vor dem Abfluss (23) oder entlang des Abflusses (23) vor dem Schwingungselement angeordnet ist.

8. Flüssigkeitsrückhaltesystem (2) nach einem der vorangehenden Ansprüche,
welches, vorzugsweise mittels einer Steuerungseinheit (26) oder mittels einer das Schwingungselement aufweisende Sensoreinheit (25), ausgebildet und eingerichtet ist, eine Information über den bestimmten Gehalt des chemischen Stoffes der Anlage (1) im wässrigen Medium (3) und/oder eine auf dem bestimmten Gehalt des chemischen Stoffes der Anlage (1) im wässrigen Medium (3) basierende Information, insbesondere eine Warnung, auszugeben und/oder auszusenden.

9. Flüssigkeitsrückhaltesystem (2) nach einem der vorangehenden Ansprüche,
welches, vorzugsweise mittels einer Steuerungseinheit (26) oder mittels einer das Schwingungselement aufweisende Sensoreinheit (25), ausgebildet und eingerichtet ist, eine Information auszugeben und/oder auszusenden, falls der betätigbare Auslass (24) geschlossen wird/ist und/oder falls ein vorbestimmter Grenzwert des bestimmten Gehalts des chemischen Stoffes der Anlage (1) im den chemischen Stoff der Anlage (1) enthaltenden wässrigen Medium (3) erreicht oder überschritten wird/ist.

10. Flüssigkeitsrückhaltesystem (2) nach einem der vorangehenden Ansprüche,
wobei das Flüssigkeitsrückhaltesystem (2), vorzugsweise eine Steuerungseinheit (26) des Flüssigkeitsrückhaltesystem (2) oder eine das Schwingungselement aufweisende Sensoreinheit (25) des Flüssigkeitsrückhaltesystem (2), ausgebildet und eingerichtet ist, das Erfassen der Resonanzfrequenz des Schwingungselements und das Bestimmen des Gehalts des chemischen Stoffes der Anlage (1) im wässrigen Medium (3) basierend auf der erfassten Resonanzfrequenz des Schwingungselements in vorbestimmten regelmäßigen zeitlichen Abständen auszuführen.

11. Flüssigkeitsrückhaltesystem (2) nach einem der vorangehenden Ansprüche,
wobei der chemische Stoff ein Gefrierschutzmittel, vorzugsweise Glykol, ist,
wobei die Anlage (1) einer Kälte- oder Klimaanlage (1) ist und
wobei das den chemischen Stoff der Anlage (1) enthaltende wässrige Medium (3) Wasser (3), vorzugsweise Kondenswasser (3) und/oder Regenwasser (3), ist.

12. System
mit einer Anlage (1), vorzugsweise einer Kälte- oder Klimaanlage (1), welche einen chemischen Stoff, vorzugsweise ein Gefrierschutzmittel, besonders vorzugsweise Glykol, enthält, und
mit einem Flüssigkeitsrückhaltesystem (2) nach einem der vorangehenden Ansprüche,
wobei die Anlage (1) zumindest abschnittsweise entlang der vertikalen Achse (Z) oberhalb der Auffangwanne (20) des Flüssigkeitsrückhaltesystem (2) angeordnet ist.
